(19) Europäisches Patentamt
European Patent Office
Office européen des brevets

(11) Veröffentlichungsnummer : **0 008 045**
**B1**

(12) # EUROPÄISCHE PATENTSCHRIFT

(45) Veröffentlichungstag der Patentschrift : **30.09.81**

(21) Anmeldenummer : **79102678.4**

(22) Anmeldetag : **27.07.79**

(51) Int. Cl.[3] : **C 07 D487/04**, C 07 D498/04, C 07 D513/04, A 61 K 31/55 // (C07D487/04, 241/00, 243/00),(C07D498/04, 265/00, 243/00),(C07D513/04, 279/00, 243/00)

(54) **(1,2)-Anellierte 7-Phenyl-1,4-benzodiazepinderivate und deren Salze; Verfahren zu deren Herstellung und diese Verbindungen enthaltende Arzneimittel.**

(30) Priorität : **16.08.78 DE 2835708**

(43) Veröffentlichungstag der Anmeldung : **20.02.80 (Patentblatt 80/04)**

(45) Bekanntmachung des Hinweises auf die Patenterteilung : **30.09.81 Patentblatt 81/39**

(84) Benannte Vertragsstaaten : **AT BE CH DE FR GB IT LU NL SE**

(56) Entgegenhaltungen :
**AT - B - 327 202**
**DE - A - 1 695 211**
**DE - A - 2 221 558**
**DE - A - 2 251 291**
**DE - A - 2 520 937**
**US - A - 3 734 912**
**US - A - 3 875 181**

(73) Patentinhaber : **Kali-Chemie Pharma GmbH**
**Hans-Böckler-Allee 20 Postfach 220**
**D-3000 Hannover 1 (DE)**

(72) Erfinder : **Liepmann, Hans, Dipl.-Chem., Dr. rer.nat.**
**Auf dem Emmerberge 17**
**D-3000 Hannover (DE)**
Erfinder : **Hüschens, Rolf, Dipl.-Chem., Dr.rer.nat.**
**Matthäikirchstrasse 25**
**D-3000 Hannover 81 (DE)**
Erfinder : **Milkowski, Wolfgang, Dipl.-Chem., Dr.rer.nat.**
**Fasanenweg 13**
**D-3167 Burgdorf (DE)**
Erfinder : **Zeugner, Horst, Dipl.-Chem., Dr.rer.nat.**
**Havelweg 10**
**D-3000 Hannover 73 (DE)**
Erfinder : **Hell, Insa, Dr. vet.**
**Wiesenstrasse 13**
**D-3000 Hannover 1 (DE)**
Erfinder : **Wolf, Klaus-Ulrich, Dr. vet.**
**Imkersweg 6**
**D-3165 Hänigsen (DE)**

(74) Vertreter : **Lauer, Dieter, Dr. et al**
**c/o Kali-Chemie Aktiengesellschaft Postfach 220**
**D-3000 Hannover 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 008 045 B1

Imprimerie Jouve, 17, rue du Louvre, 75001 Paris, France

# [1,2]-Anellierte 7-Phenyl-1,4-benzodiazepinderivate und deren Salze ; Verfahren zu deren Herstellung und diese Verbindungen enthaltende Arzneimittel

Die Erfindung betrifft [1,2]-anellierte 7-Phenyl-1,4-benzodiazepinderivate, deren pharmakologisch verträgliche Salze, Verfahren zu deren Herstellung sowie diese Substanzen enthaltende pharmazeutische Zusammensetzungen.

Bekanntlich zeigen 5-Phenyl-1,4-benzodiazepinderivate, wie beispielsweise 5-Phenyl-1,4-benzodiazepin-2-one, durch ihre ZNS-Wirksamkeit auch einen gewissen streßulkushemmenden Effekt. Die Wirkung reicht aber nicht aus, um diese Substanzen für die wirksame Behandlung von Patienten einzusetzen.

Überraschenderweise wurde nun gefunden, daß die erfindungsgemäßen [1,2]-anellierten 7-Phenyl-1,4-benzodiazepinderivate nur eine verhältnismäßig geringe ZNS-Wirksamkeit besitzen, dafür aber eine gute ulkushemmende Wirkung bei Ulkusleiden verschiedenen Ursprungs zeigen, so daß sie sich für die Ulkustherapie des Magens und des Duodenum eignen.

Gegenstand der Erfindung sind allgemeinen [1,2]-anellierte 7-Phenyl-1,4-benzodiazepine der Formel I

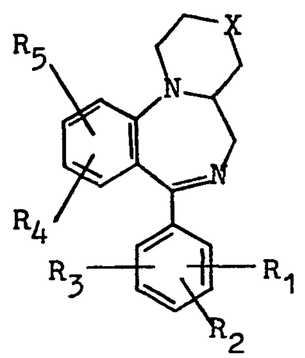

(I)

worin X ein Sauerstoffatom, ein Schwefelatom oder eine Iminogruppe NR ist, in welcher R ein Wasserstoffatom, ein $C_1$-$C_5$-Alkylrest, ggf. endständig substituiert mit einem Phenylrest, der durch 1 oder 2 Methoxy oder durch 3,4-Methylen- oder 3,4-Äthylendioxy substituiert sein kann, ein $C_2$-$C_5$-Alkyl, endständig substituiert mit Halogen, Hydroxy oder Methoxy, oder ein $C_3$-$C_5$-Alkenylrest ist, $R_1$ bis $R_5$ gleich oder verschieden sind und ein Wasserstoffatom, ein Halogenatom, eine Trifluormethylgruppe, eine Nitrogruppe, eine Alkylgruppe, eine Alkoxygruppe oder eine Alkylthiogruppe bedeuten, wobei der Alkylrest jeweils 1 bis 4 C-Atome haben kann, oder zwei benachbarte Reste eine Methylen- oder Äthylendioxygruppe sind sowie deren Säureadditionssalze.

Als Alkyl- oder Alkenylreste am Stickstoffatom kommen gerade oder verzweigte Gruppen mit 1 bis 5 C-Atomen bzw. 3 bis 5 C-Atomen in Frage, so beispielsweise Methyl-, Äthyl-, Propyl-, Isopropyl-, Butyl-, Isobutyl-, tert.-Butyl-, Pentyl-, Isopentyl-, Neopentyl-, Allyl-, 2- oder 3-Butenyl-, 2-, 3- oder 4-Pentenylreste. Bevorzugt werden die Methylgruppe und die Äthylgruppe, die mit einem Phenylrest endständig substituiert sind, sowie die mit Chlor, Hydroxy oder Methoxy endständig substituierte Äthyl- oder Propylgruppe.

Für die Substituenten $R_1$ bis $R_5$ an den Phenylringen kommen als Halogenatome Fluor-, Chlor-, Brom- oder Jodatome, insbesondere Fluor-, Chlor- oder Bromatome, in Frage. Die $C_1$-$C_4$-Alkylreste in den Alkyl-, Alkoxy- oder Alkylthiogruppen können gerade oder verzweigt sein, wobei insbesondere bei Mehrfachsubstitution an dem jeweiligen Phenylrest der Methylrest, so Methyl, Methoxy, Methylthio oder Methylendioxy vorherrscht.

Bei Mono- oder Disubstitution am Phenylring des 1,4-Benzodiazepingerüstes befinden sich $R_4$ und $R_5$ vorzugsweise in 9- und/oder 10-Stellung. Bei Substitution durch Fluor, Nitrogruppe oder Trifluormethylgruppe ist die Einfachsubstitution in 9-Stellung bevorzugt, für Methoxy und Methylthio die in 10-Stellung.

Bei Einfachsubstitution ist am 7-Phenylrest Halogen, Methyl, Methoxy oder Trifluormethyl in 2'- oder 3'-Stellung bevorzugt. Bei Mehrfachsubstitution durch gleiche oder verschiedene Substituenten $R_1$ bis $R_3$ sind die 3',4'- oder 3',4',5'-Stellungen bevorzugt. In Frage kommen Halogenatome, insbesondere Chloratome, Methyl-, Methoxygruppen und/oder die Methylen- oder Äthylendioxygruppe.

Die erfindungsgemäßen Verbindungen und ihre Salze weisen wertvolle therapeutische Eigenschaften auf, insbesondere zeigen sie eine ausgeprägte ulkushemmende Wirkung. Es ist bekannt, daß die Ätiologie des Ulkusleidens sehr komplexer Natur ist. Da mit den bisher gebräuchlichen Pharmaka jeweils Pnur Teilaspekte dieses vielschichtigen Geschehens beeinflußt werden, konnten auch nur begrenzte Erfolge erzielt werden (s. Blum, Schweiz. Med. Wochenschrift, 106 [1976] S. 1457).

Nach Demling (L. Demling, Klin. Gastroenterologie I, [1973], S. 202) ist im Falle der Magen- und Darmulzeration das Gleichgewicht der auf die Schleimhaut einwirkenden aggressiven und defensiven Faktoren gestört. Eine Therapie muß daher darauf ausgerichtet sein, dieses Gleichgewicht wieder herzustellen.

Es ist bekannt, daß Psychopharmaka, insbesonders 5-Phenyl-1,4-benzodiazepin-2-on-derivate, eine streßabschirmende Wirkung haben und somit einen gewissen streßulkushemmenden Effekt aufweisen. Diese Psychopharmaka haben sich in der Ulkustherapie aber wegen zu geringer Wirkung bei vertretbarer Dosierung nicht durchsetzen können. Ihre ZNS-Wirkungen, wie Sedierung und Beeinflussung des Muskeltonus, sind zudem in der ambulanten Therapie unerwünscht.

Die erfindungsgemäßen [1,2]-anellierten 1,4-Benzodiazepinderivate zeigen demgegenüber neben der durch Streß bedingten Beeinflussung am Ulkus eine überraschend gute Wirkung am Ulkus anderer Pathogenese, so u.a. am durch Arzneimittel induzierten Ulkus, wie beispielsweise dem durch Indomethacin verursachten Ulkus. Bemerkenswert ist bei den vorliegenden Substanzen das Fehlen der ausgeprägten ZNS-Wirkung der handelsüblichen 1,4-Benzodiazepin-2-on-derivate, so daß bei Gabe von therapeutischen Mengen an erfindungsgemäßen Verbindungen oder deren Säureadditionssalzen mit störenden Nebenwirkungen, wie beispielsweise Sedation oder Beeinflussung des Muskeltonus, nicht zu rechnen ist. Infolge ihrer geringen Toxizität zeigen die Substanzen zudem eine gute therapeutische Breite. Sie eignen sich daher insbesondere für den Einsatz in der ambulanten Ulkusbehandlung.

Beschreibung der pharmakologischen Untersuchungsmethoden

1. Akute Toxizität

Die akute 7-Tage-Toxizität wird nach einmaliger Applikation per os an der weißen nüchternen NMRI-Maus bestimmt. Die Berechnung der $LD_{50}$-Werte erfolgt über EDV durch eine Probitanalyse (L. Cavalli-Sforza, Gustav-Fischer-Verlag, Stuttgart [1964], Grundbegriffe der Biometrie, Kapitel 10, S. 153-190).

2. Prüfung am Indomethacinulkus der Ratte (Modifizierte Versuchsanordnung nach U. Jahn und R. W. Adrian, Arzneim. Forsch. (Drug Res.) 19, (1969), S. 36).

Die Prüfsubstanz wird mindestens 6 männlichen Ratten von 155 bis 190 g Körpergewicht in 0,5 ml Suspensionsmedium/100 g Tiergewicht per os appliziert. Die Tiere der Kontrollgruppe erhalten das entsprechende Volumen des Suspensionsmediums. Eine Stunde nach der Applikation werden den Ratten p.o. 40 mg/kg Indomethacin in 0,5 ml Suspensionsmedium/100 g Tiergewicht zur Erzeugung von Ulzera verabreicht. Die Tiere werden 24 h nach der Indomethacin-Applikation getötet.

Die Auswertung erfolgt modifiziert nach O. Münchow, (Arzneim. Forsch. (Drug Res.) *4*, [1954] S. 341 344). Es werden Mittelwert und Standardabweichung der Ulkuszahlen berechnet und anschließend die Hemmwirkung von Test- und Standard-Substanz in Prozent gegenüber der Kontrolle bestimmt.

3. Prüfung der muskulotropen Eigenschaften

Im Test de la Traction wird Mäusen die Versuchssubstanz per os verabreicht. Nach 120 Minuten werden die Mäuse mit den Vorderpfoten an einen dünnen waagerecht gespannten Draht gehängt. Als $ED_{50}$ gilt die Dosis, bei der gerade die Hälfte der Tiere nicht innerhalb von 5 sec auch mit den Hinterpfoten den Draht berührt (W. Theobald et al. Arzneim. Forsch. 17, 561 [1967]). In diesem Test wird die Beeinflussung des Muskeltonus durch die Prüfsubstanzen getestet.

4. Prüfung auf zentraldämpfende Eingenschaften (Verlängerung der Hexobarbitalschlafzeit)

Die Prüfsubstanz wird den Mäusen per os appliziert. Nach 30 Minuten erhalten die Tiere zusätzlich eine i.v. Injektion von 64 mg/kg Hexobarbital. Der Zeitpunkt der Einnahme der Seitenlage wird festgestellt und die Dauer der Seitenlage mit einer lediglich mit Hexobarbital behandelten Kontrollgruppe verglichen. Als $ED_{50}$ ist eine Dosis definiert, bei der die Hälfte der Tiere eine um den Faktor 4 verlängerte Seitenlage gegenüber der Kontrollgruppe beibehält (J. W. Kemp, M. Tannhauser und E. A. Swinyard, Arch. int. Pharmacodyn. 193 [1971], 37-47).

Folgende Verbindungen wurden nach den vorstehend beschriebenen Methoden untersucht :

A) 1,2,4,4a-Tetrahydro-9-chlor-7-phenyl-5H[1,4]oxazino[4,3-a][1,4]benzodiazepin-maleinat

B) 1,2,4,4a-Tetrahydro-9-fluor-7-phenyl-5H[1,4]oxazino[4,3-a][1,4]benzodiazepin

C) 1,2,4,4a-Tetrahydro-9-methyl-7-phenyl-5H[1,4]oxazino[4,3-a][1,4]benzodiazepin

D) 1,2,4,4a-Tetrahydro-10-methyl-7-phenyl-5H[1,4]oxazino[4,3-a][1,4]benzodiazepin

E) 1,2,4,4a-Tetrahydro-9-chlor-5-(2'-chlorphenyl)-5H[1,4]oxazino[4,3-a][1,4]benzodiazepin

F) 1,2,4,4a-Tetrahydro-10-methoxy-7-phenyl-5H[1,4]-oxazino[4,3-a][1,4]benzodiazepin

G) 1,2,4,4a-Tetrahydro-10-chlor-9-methyl-7-phenyl-5H[1,4]oxazino[4,3-a][1,4]benzodiazepin

H) 1,2,4,4a-Tetrahydro-9-chlor-7-phenyl-5H[1,4]thiazino[4,3-a][1,4]benzodiazepin

I) 1,2,3,4,4a,5-Hexahydro-9-chlor-3-allyl-7-phenyl-pyrazino[1,2-a][1,4]benzodiazepin-dihydroclorid 1,5 Mol $H_2O$ - 0,3 Mol Isopropranol

K) 1,2,3,4,4a,5-Hexahydro-9-chlor-3-phenäthyl-7-phenyl-pyrazino[1,2-a][1,4]benzodiazepin

L) 1,2,3,4,4a,5-Hexahydro-3,9-dimethyl-7-phenyl-pyrazino[1,2-a][1,4]benzodiazepin-dihydrochlorid 1,5 Mol $H_2O$

S) Standard : 7-Chlor-1-methyl-5-phenyl-1,3-dihydro-2H-1,4-benzodiazepin-2-on (Diazepam)

Tabelle

| Sub-stanz | $LD_{50}$ p.o. (mg/kg) | Ulkus, induziert durch Indomethacin p.o. (mg/kg) | % Hemmung | Test de la Traction $ED_{50}$ (mg/kg) | 4-fache Verlängerg der Hexo-barbital-schlafzeit (mg/kg) |
|---|---|---|---|---|---|
| A | 2260 | 150 | 61 | 285,0 | 31,6 |
| B | >1370 | 100 | 77 | > 296 | 80,3 |
| C | >1430 | 200 | 68 | > 316 | 139 |
| D | >1470 | 100 | 70 | > 316 | 138 |
| E | >1470 | 100 | 86 | > 316 | 11,8 |
| F | >1430 | 100 | 42 | > 308 | 202 |
| G | >1520 | 200 | 77 | > 327 | 119 |
| H | >1470 | 100 | 74 | 316 | 153 |
| I | 1090 | 200 | 81 | > 316 | 100 |
| K | >1310 | 200 | 85 | > 284 | 110 |
| L | 1010 | 250 | 70 | >215 | 164 |
| S | 887 | 12*) | 28 | 4,2 | 1,5 |

*) Der Standard Diazepam zeigt bereits bei niedriger Dosierung eine starke psychopharmakologische Wirkung, so daß eine höhere Dosierung zur Erzielung einer besseren Ulkushemmung nicht tolerierbar ist.

Aus den gefundenen Werten geht eindeutig hervor, daß die erfindungsgemäßen Substanzen bei geringer ZNS-Wirkung eine gute Ulkuswirkung aufweisen.

Die Verbindungen der allgemeinen Formel I und ihre Salze lassen sich in bekannter Weise in die üblichen pharmazeutischen Zubereitungsformen, z.B. in Lösungen, Suppositorien, Tabletten, Kapseln oder Dragées, einarbeiten. Die Einzeldosis beträgt für Erwachsene bei oraler Applikation 50 bis 150 mg und die Tagesdosis 150 bis 450 mg.

Die erfindungsgemäßen Verbindungen der allgemeinen Formel I

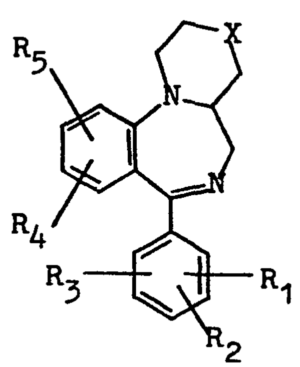

(I)

in welcher X und $R_1$ bis $R_5$ die obige Bedeutung haben, werden dadurch hergestellt, daß man Verbindungen der allgemeinen Formel II

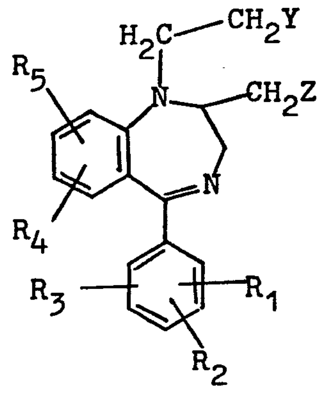

(II)

in welcher $R_1$ bis $R_5$ die obige Bedeutung haben, und Y und Z gleich oder verschieden sind und einen reaktiven Rest darstellen, so ein Halogen, insbesondere Chloroder Brom, einen Toluolsulfonyloxy-, Benzolsulfonyloxy- oder Methansulfonyloxyrest, in Gegenwart eines Alkalimetall- oder Erdalkalimetall-hydroxids, Alkalimetall- oder Erdalkalimetallcarbonats, Alkalimetall- oder Erdalkalimetallsulfids oder einer Aminoverbindung $RNH_2$, wobei R die obige Bedeutung hat, oder des entsprechenden Alkalimetallamids bei Temperaturen zwischen 60 und 120 °C, ggf. in Gegenwart eines inerten Lösungsmittels, cyclisiert und ggf. die so gebildete Base in das Säureadditionssalz überführt oder aus dem Säureadditionssalz die freie Base isoliert.

Vorzugsweise werden Verbindungen der allgemeinen Formel II als Ausgangsmaterialien herangezogen, in welchen Y und Z die gleiche Bedeutung haben. Besonders günstig ist der Einsatz von Verbindungen der allgemeinen Formel II, in welchen Y und Z chloratome bedeuten. Werden Verbindungen der allgemeinen Formel II herangezogen, in welchen Y und Z Sulfonyloxyreste bedeuten, so hat sich insbesondere der Toluolsulfonyloxyrest bewährt. Die Verbindungen lassen sich in an sich bekannter Weise aus den Verbindungen der allgemeinen Formel III

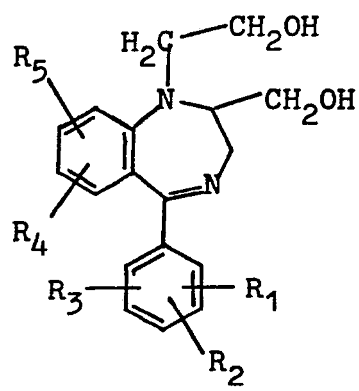

(III)

in welcher $R_1$ bis $R_5$ die obige Bedeutung haben, durch Umsetzung mit den entsprechenden Sulfonylchloriden, beispielsweise p-Toluolsulfonylchlorid, in Gegenwart eines inerten Lösungsmittels herstellen. Dabei ist es nicht erforderlich, daß man die gebildeten Sulfonsäureester vor der weiteren Umsetzung isoliert.

Zur Herstellung der anellierten Oxazino- und Thiazino[4,3-a][1,4]benzodiazepinderivate I werden die Verbindungen der allgemeinen Formel II vorzugsweise mit Hydroxiden, Carbonaten oder Sulfiden des Natriums, Kaliums, Calciums oder Bariums umgesetzt. Im allgemeinen wird es vorgezogen, die Cyclisierung in Gegenwart eines organischen Lösungsmittels, beispielsweise von niederen Alkoholen, Aceton, Diäthyläther, Dioxan, Tetrahydrofuran, Pyridin, Dimethylsulfoxid oder Dimethylformamid, vorzunehmen, wobei die Zugabe von Wasser vorteilhaft sein kann. Begünstigt wird die Reaktion, wenn die Reaktionsteilnehmer in Lösung vorliegen.

Bei der Herstellung der anellierten Pyrazino[1,2-a][1,4]-benzodiazepine können die gleichen Lösungsmittel eingesetzt werden, es ist aber auch möglich, das primäre organische Amin als Lösungsmittel heranzuziehen.

Die Umsetzung kann bei Normaldruck oder erhöhtem Druck durchgeführt werden. Insbesondere bei Cyclisierung in Gegenwart von Ammoniak oder niedrig siedenden Aminen arbeitet man im geschlossenen Gefäß.

Die nachträgliche Substitution durch Halogen oder die Nitrogruppe im Phenylring des 1,4-Benzodiazepinsystems ist in an sich bekannter Weise möglich. Als Halogenierungsmittel können beispielsweise N-Chlorsuccinimid oder N-Bromsuccinimid dienen. Zur Einführung der Nitrogruppe können die üblichen Nitrierungsreagenzien herangezogen werden, beispielsweise Kupfer(II)-nitrat-trihydrat in Acetanhydrid.

Weiter ist es möglich, in den 1,2,3,4,4a,5-Hexahydro-7-phenyl-pyrazino[1,2-a][1,4]benzodiazepinderivaten der allgemeinen Formel I die NH-Gruppe nachträglich in an sich bekannter Weise zu alkylieren. Übliche Methoden sind die Umsetzung dieser Verbindungen mit Halogenalkylen (s. Houben-Weyl, Bd. XI/1, [1957] S. 24 ff.), mit Dialkylsulfat, beispielsweise Dimethyl- oder Diäthylsulfat oder Äthylendisulfat (s.a.a. O.S. 207), mit Sulfonsäureestern der Formel R'-$SO_3$R, in welcher R' beispielsweise Methyl-Phenyl- oder 4-Methylphenyl und R eine Alkylgruppe ist (s.a.a. O.S. 217) oder mit Äthylen- und Propylenoxid (s.a.a. O.S. 311).

Weiter ist es möglich, in den 1,2,3,4,4a,5-Hexahydro-3-(hydroxyalkyl)-7-phenyl-pyrazino[1,2-a][1,4]benzodiazepinen der allgemeinen Formel I nachträglich die Hydroxyalkylgruppe in die Methoxyalkylgruppe (s. Houben-Weyl, Bd. VI/3 [1965] S. 24) oder in die Halogenalkylgruppe (s.a.a. O. Bd. V/3, [1962] S. 862) überzuführen.

Die Herstellung der 1,4-Benzodiazepinderivate der allgemeinen Formel II, in welcher $R_1$ bis $R_5$ die obige Bedeutung haben und Y und Z Halogenatome sind, sowie die der 1,4-Benzodiazepinderivate der allgemeinen Formel III, in welcher $R_1$ bis $R_5$ die obige Bedeutung haben, ist in der DE-OS 22 21 558 sowie DE-PS 25 20 937 beschrieben. Als Grundreaktion ist die Cyclisierung von Acyldiaminen mit Phosphoroxychloriden bei Temperaturen zwischen etwa 90 und 130 °C anzusehen ; es werden dabei entweder direkt die 1,4-Benzodiazepine der allgemeinen Formel II erhalten oder Gemische aus 1,5-Benzodiazocinen und 1,4-Benzodiazepinen, die anschließend durch Behandlung mit nukleophilen Reagenzien in 1,4-Benzidiazepinderivate umgelagert werden können.

Die nachstehenden Beispiele dienen zur Erläuterung der Erfindung :

## Beispiel 1

Eine Lösung von 20 g (7-Chlor-1-(β-chloräthyl)-ç-chlormethyl-5-phenyl-2,3-dihydro-1H-1,4-benzodiazepin in 100 ml Dioxan und 250 ml 6%-iger Natriumhydroxidlösung wird 5,5 h unter Rückfluß erhitzt. Nach Abziehen des Lösungsmittels im Vakuum wird die Substanz aus Chloroform isoliert, anschließend an Aluminiumoxid der Aktivitätsstufe II mit Methylenchlorid chromatographiert. Es werden 12,8 g 1,2,4,4a-Tetrahydro-9-chlor-7-phenyl-5H[1,4] oxazino[4,3-a][1,4]benzodiazepin als Öl erhalten. Das Maleinat kristallisiert aus Isopropanol/Äther mit Fp. 143-145 °C. Die aus Hexan kristallisierte Base schmilzt bei 156-158 °C.

In gleicher Weise erhält man aus dem 1-(β-Chloräthyl)-2-chlormethyl-5-phenyl-2,3-dihydro-1H-1,4-benzodiazepin sowie dessen entsprechenden 7-Fluor-, 7-Brom-, 7-Nitro-, 7-Methyl-, 8-Methyl-, 7-Trifluormethyl-, 7,8-Dimethyl-, 8-Methoxy-, 7,8-Dichlor-, 8-Chlor-7-methyl-, 7-Brom-8-methyl-, 7,8-Methylendioxy-derivaten oder den 7-Chlor-1-(β-chloräthyl)-2-chlormethyl-5-[(3'-trifluormethylphenyl)-bzw. (3',4'-dichlorphenyl)]-2,3-dihydro-1H-1,4-benzodiazepinen oder dem 7-Fluor-1-(β-chloräthyl)-2-chlormethyl-5-(3',4',5'-trimethoxyphenyl)-2,3-dihydro-1H-1,4-benzodiazepin die folgenden Verbindungen :

| | Fp. °C |
|---|---|
| 1,2,4,4a-Tetrahydro-7-phenyl-5H[1,4]oxazino-[4,3-a][1,4]benzodiazepin | 102-105 |
| 1,2,4,4a-Tetrahydro-9-fluor-7-phenyl-5H-[1,4]oxazino[4,3-a][1,4]benzodiazepin | 154-156 |
| 1,2,4,4a-Tetrahydro-9-brom-7-phenyl-5H-[1,4]oxazino[4,3-a][1,4]benzodiazepin | 176-179 |
| 1,2,4,4a-Tetrahydro-9-nitro-7-phenyl-5H-[1,4]oxazino[4,3-a][1,4]benzodiazepin | 142-145 |
| 1,2,4,4a-Tetrahydro-9-methyl-7-phenyl-5H-[1,4]oxazino[4,3-a][1,4]benzodiazepin | 174-176 |

| | Fp. °C |
|---|---|
| 1,2,4,4a-Tetrahydro-10-methyl-7-phenyl-5H-[1,4]oxazino[4,3-a][1,4]benzodiazepin | 123-124 |
| 1,2,4,4a-Tetrahydro-9-trifluormethyl-7-phenyl-5H-[1,4]oxazino[4,3-a][1,4]benzodiazepin<br>IR: 1615 $cm^{-1}$ (C=N) | Öl |
| 1,2,4,4a-Tetrahydro-9,10-dimethyl-7-phenyl-5H[1,4]oxazino[4,3-a][1,4]benzodiazepin | 132-134 |
| 1,2,4,4a-Tetrahydro-10-methoxy-7-phenyl-5H-[1,4]oxazino[4,3-a][1,4]benzodiazepin | 107-110 |
| 1,2,4,4a-Tetrahydro-9,10-dichlor-7-phenyl-5H[1,4]oxazino[4,3-a][1,4]benzodiazepin | 163-165 |
| 1,2,4,4a-Tetrahydro-10-chlor-9-methyl-7-phenyl-5H[1,4]oxazino[4,3-a][1,4]benzodiazepin | 115-118 |
| 1,2,4,4a-Tetrahydro-9-brom-10-methyl-7-phenyl-5H[1,4]oxazino[4,3-a][1,4]benzodiazepin | 117-119 |
| 1,2,4,4a-Tetrahydro-9,10-methylendioxy-7-phenyl-5H[1,4]oxazino[4,3-a][1,4]benzodiazepin | 156-158 |
| 1,2,4,4a-Tetrahydro-9-chlor-7-(3'-trifluormethylphenyl-5H[1,4]oxazino[4,3-a][1,4]benzodiazepin | 104-107 |
| 1,2,4,4a-Tetrahydro-9-chlor-7-(3',4'-dichlorphenyl-5H[1,4]oxazino[4,3-a][1,4]benzodiazepin | 145-148 |
| 1,2,4,4a-Tetrahydro-9-fluor-7-(3',4',~~4'~~,5'-trimethoxyphenyl)-5H[1,4]oxazino[4,3-a][1,4]-benzodiazepin | 165-166 |

## Beispiel 2

Zu einer gerührten Suspension von 8,0 g 7-Chlor-1-(β-hydroxyäthyl)-2-hydroxymethyl-5-(2'-chlorphenyl)-2,3-dihydro-1H-1,4-benzodiazepin und 5,2 g p-Toluolsulfochlorid in 80 ml Dioxan wird eine Lösung von 5,2 g Kalium-hydroxid in 17,2 ml Wasser gegeben und anschließend 1 h unter Rückfluß erhitzt. Die organische Phase wird von der wäßrigen Phase abgetrennt und im Vakuum abgezogen. Das Reaktionsprodukt wird aus Chloroform isoliert. Nach Chromatographie an Aluminiumoxid der Aktivitätsstufe II mit Chloroform werden 4,8 g 1,2,4,4a-Tetrahydro-9-chlor-7-(2'-chlorphenyl)-5H[1,4]oxazino[4,3-a][1,4]benzodiazepin mit Fp. 140-141 °C erhalten.

Entsprechend erhält man aus

1-(β-Hydroxyäthyl-2-hydroxymethyl-5-[(2'-chlorphenyl)- bzw. (2'-fluorphenyl)]-2,3-dihydro-1H-1,4-benzodiazepin

1,2,4,4a-Tetrahydro-7-(2'-chlorphenyl)-5H[1,4]oxazino[4,3-a][1,4]benzodiazepin als Ol. IR: 1 615 $cm^{-1}$ (C = N).

1,2,4,4a-Tetrahydro-7-(2'-fluorphenyl)-5H-[1,4]oxazino[4,3-a][1,4]benzodiazepin als Öl. IR: 1 610 $cm^{-1}$ (C = N).

## Beispiel 3

10,0 g 7-Chlor-1-(β-chloräthyl)-2-chlormethyl-5-phenyl-2,3-dihydro-1H-1,4-benzodiazepin werden in 100 ml Äthanol mit 7,2 g Natriumsulfid-nonahydrat 4 h unter Rückfluß erhitzt. Das Lösungsmittel wird im Vakuum abgezogen und das Reaktionsprodukt aus Chloroform isoliert. Nach chromatographischer Reinigung an Aluminiumoxid der Aktivitätsstufe II mit Methylenchlorid werden durch Kristallisation aus Hexan 5,4 g 1,2,4,4a-Tetrahydro-9-chlor-7-phenyl-5H[1,4]thiazino[4,3-a][1,4]benzodiazepin mit Fp. 136-138 °C erhalten.

In gleicher Weise erhält man aus dem 1-(β-Chloräthyl)-2-chlormethyl-5-phenyl-2,3-dihydro-1H-1,4-benzodiazepin, dessen 7-Fluor-, 7-Nitro-, 7-Methyl- und 8-Methoxy-derivaten bzw. dessen 2'-Fluorphenylderivat sowie 7-Chlor-1-(β-chloräthyl)-2-chlormethyl-5-(3'-trifluormethylphenyl)-2,3-dihydro-1H-1,4-benzodiazepin.

Die folgenden Verbindungen:

| | Fp. °C |
|---|---|
| 1,2,4,4a-Tetrahydro-7-phenyl-5H/1,4/thiazino-/4,3-a//1,4/benzodiazepin | 125-128 |
| 1,2,4,4a-Tetrahydro-9-fluor-7-phenyl-5H-/1,4/thiazino/4,3-a//1,4/benzodiazepin | 168-170 |
| 1,2,4,4a-Tetrahydro-9-nitro-7-phenyl-5H-/1,4/thiazino/4,3-a//1,4/benzodiazepin | 166-172 |
| 1,2,4,4a-Tetrahydro-9-methyl-7-phenyl-5H-/1,4/thiazino/4,3-a//1,4/benzodiazepin | 143-146 |
| 1,2,4,4a-Tetrahydro-10-methoxy-7-phenyl-5H-/1,4/thiazino/4,3-a//1,4/benzodiazepin | 125-131 |
| 1,2,4,4a-Tetrahydro-7-(2'-fluorphenyl)-5H-/1,4/thiazino/4,3-a//1,4/benzodiazepin IR: 1610 $cm^{-1}$ (C=N) | Öl |
| 1,2,4,4a-Tetrahydro-9-chlor-7-(3'-trifluormethylphenyl)-5H/1,4/thiazino/4,3-a//1,4/benzodiazepin | 102-104 |

## Beispiel 4

10 g 7-Chlor-1-(β-chloräthyl)-2-chlormethyl-5-phenyl-2,3-dihydro-1H-1,4-benzodiazepin werden in 200 ml Methanol mit 20 g Ammoniakgas im Glasautoklav 16 h bei 90 bis 95 °C umgesetzt. Nach Abziehen

des Lösungsmittels im Vakuum wird das Reaktionsgemisch in Chloroform aufgenommen und mit verdünnter wäßriger Natriumhydroxidlösung gewaschen. Nach Trocknen und Abziehen des Lösungsmittels im Vakuum wird das erhaltene ölige Reaktionsprodukt in Äther aufgenommen. Aus der konzentrierten Lösung kristallisieren 5 g 1,2,3,4,4a,5-Hexahydro-9-chlor-7-phenyl-pyrazino[1,2-a][1,4]benzodiazepin mit Fp. 132-135 °C.

In gleicher Weise erhält man aus dem 1-(β-Chloräthyl)-2-chlormethyl-5-phenyl-2,3-dihydro-1H-1,4-benzodiazepin, dessen 7-Brom-, 7-Methyl-, 8-Methoxy-, 7,8-Dimethyl- und 7-Brom-8-methyl-derivaten, dem 7-Chlor-1-(β-chloräthyl)-2-chlormethyl-5-(2'-chlorphenyl)- bzw. 7-Chlor- oder 7-Brom-1-(β-chloräthyl)-2-chlormethyl-5-(3',4'-dichlorphenyl)-2,3-dihydro-1H-1,4-benzodiazepin.

Die folgenden Verbindungen :

| | Fp. °C |
|---|---|
| 1,2,3,4,4a,5-Hexahydro-7-phenyl-pyrazino-/1,2-a/7/1,4/7benzodiazepin. IR: 3500 $cm^{-1}$ (NH)<br>1610 $cm^{-1}$ (C=N) | Öl |
| 1,2,3,4,4a,5-Hexahydro-9-brom-7-phenyl-pyrazino/1,2-a/7/1,4/7benzodiazepin | 133-138 |
| 1,2,3,4,4a,5-Hexahydro-9-methyl-7-phenyl-pyrazino/1,2-a/7/1,4/7benzodiazepin-ditosylat | 192-198 |
| 1,2,3,4,4a,5-Hexahydro-10-methoxy-7-phenyl-pyrazino/1,2-a/7/1,4/7benzodiazepin.<br>IR: 3500 $cm^{-1}$ (NH)<br>1610 $cm^{-1}$ (C=N) | Öl |
| 1,2,3,4,4a,5-Hexahydro-9,10-dimethyl-7-phenyl-pyrazino/1,2-a/7/1,4/7benzodiazepin-ditosylat | 232-234 |
| 1,2,3,4,4a,5-Hexahydro-9-brom-10-methyl-7-phenyl-pyrazino/1,2-a/7/1,4/7benzodiazepin-ditosylat | 260-263 |
| 1,2,3,4,4a,5-Hexahydro-9-chlor-7-(2'-chlorphenyl)-pyrazino/1,2-a/7/1,4/7benzodiazepin | 131-137 |
| 1,2,3,4,4a,5-Hexahydro-9-chlor-7-(3',4'-dichlorphenyl)-pyrazino/1,2-a/7/1,4/7benzodiazepin<br>IR: 3500 $cm^{-1}$ (NH)<br>1610 $cm^{-1}$ (C=N) | Öl |
| 1,2,3,4,4a,5-Hexahydro-9-brom-7-(3',4'-dichlorphenyl)-pyrazino/1,2-a/7/1,4/7benzodiazepin-dihydrochlorid . 1 Mol Isopropanol . o,3 Mol $H_2O$ | 210-212 |

Beispiel 5

18,4 g 7-Chlor-1-(β-chloräthyl)-2-chlormethyl-5-phenyl-2,3-dihydro-1H-1,4-benzodiazepin werden in 200 ml Methanol mit 10 g Methylamin 16 h bei 110 °C im Glasautoklav umgesetzt. Nach Aufarbeitung des Reaktionsproduktes erhält man 12 g 1,2,3,4,4a,5-Hexahydro-9-chlor-3-methyl-7-phenyl-pyrazino[1,2-a][1,4] benzodiazepindihydrochlorid·1 Mol $H_2O$ mit Fp. 262 °C (unter Zersetzung). In entsprechender Weise erhält man aus 7-Chlor-1-(β-chloräthyl)-2-chlormethyl-5-phenyl-2,3-dihydro-1H-1,4-benzodiazepin in Methanol mit a) Isopropylamin, b) n-Butylamin, c) tert.-Butylamin, d) Allylamin, e) Äthanolamin und f) β-(3,4-Dimethoxyphenyl)-äthylamin. folgende Verbindungen :

| | Fp. °C |
|---|---|
| a) 1,2,3,4,4a,5-Hexahydro-9-chlor-3-isopropyl-7-phenyl-pyrazino/1,2-a7/1,47benzodiazepin-dihydrochlorid · 2,5 Mol $H_2O$ | 236 - 245 |
| b) 1,2,3,4,4a,5-Hexahydro-9-chlor-3-butyl-7-phenyl-pyrazino/1,2-a7/1,47benzodiazepin | 104 - 106 |
| c) 1,2,3,4,4a,5-Hexahydro-9-chlor-3-tert.-butyl-7-phenyl-pyrazino/1,2-a7/1,47benzodiazepin | 143 - 146 |
| d) 1,2,3,4,4a,5-Hexahydro-9-chlor-3-allyl-7-phenyl-pyrazino/1,2-a7/1,47benzodiazepin-dihydrochlorid · 1,5 Mol $H_2O$ · 0,3 Mol Isopropanol | 230 - 233 |
| e) 1,2,3,4,4a,5-Hexahydro-9-chlor-3-(ß-hydroxyäthyl)-7-phenyl-pyrazino/1,2-a7/1,47benzodiazepin | 129 - 131 |
| als Dihydrochlorid · 2,5 Mol $H_2O$ | 216 |
| f) 1,2,3,4,4a,5-Hexahydro-9-chlor-3-(3,4-dimethoxyphenäthyl)-7-phenyl-pyrazino/1,2-a7/1,47 benzodiazepin-dihydrochlorid-dihydrat | 180 |

Beispiel 6

Wird Methylamin in methanolischer Lösung bei Temperaturen zwischen 90 und 95 °C im Glasautoklav umgesetzt mit

a) 7-Chlor-1-(β-chloräthyl)-2-chlormethyl-5-(2'-chlorphenyl)-2,3-dihydro-1H-1,4-benzodiazepin
b) 7-Chlor-1-(β-chloräthyl)-2-chlormethyl-5-(2'-methoxyphenyl)-2,3-dihydro-1H-1,4-benzodiazepin
c) 7-Methyl-1-(β-chloräthyl)-2-chlormethyl-5-phenyl-2,3-dihydro-1H-1,4-benzodiazepin
d) 8-Methyl-1-(β-chloräthyl)-2-chlormethyl-5-phenyl-2,3-dihydro-1H-1,4-benzodiazepin
e) 8-Methyl-1-(β-chloräthyl)-2-chlormethyl-5-(2'-chlorphenyl)-2,3-dihydro-1H-1,4-benzodiazepin
f) 7-Brom-8-methyl-1-(β-chloräthyl)-2-chlormethyl-5(2'-chlorphenyl)-2,3-dihydro-1H-1,4-benzodiazepin
g) 7,8-Dimethyl-1-(β-chloräthyl)-2-chlormethyl-5-phenyl-2,3-dihydro-1H-1,4-benzodiazepin
h)-m) 1-(β-Chloräthyl)-2-chlormethyl-5-phenyl-2,3-dihydro-1H-1,4-benzodiazepin sowie dessen 7-Fluor-, 7-Nitro-, 7,8-Dichlor- und 8-Methoxy-derivate,
n) 1-(β-Chloräthyl)-2-chlormethyl-5-(2'-chlorphenyl)-2,3-dihydro-1,4-benzodiazepin

so erhält man :

| | Fp. °C |
|---|---|
| a) 1,2,3,4,4a,5-Hexahydro-9-chlor-3-methyl-7-(2'-chlorphenyl)-pyrazino[1,2-a][1,4]-benzodiazepin | 137 - 138 |
| b) 1,2,3,4,4a,5-Hexahydro-9-chlor-3-methyl-7-(2'-methoxyphenyl)-pyrazino[1,2-a][1,4]-benzodiazepin; IR: 1615 $cm^{-1}$ (C=N) | Öl |
| c) 1,2,3,4,4a,5-Hexahydro-3,9-dimethyl-7-phenyl-pyrazino[1,2-a][1,4]benzodiazepin-dihydrochlorid · 1,5 Mol $H_2O$ | 230 - 233 |
| d) 1,2,3,4,4a,5-Hexahydro-3,10-dimethyl-7-phenyl-pyrazino[1,2-a][1,4]benzodiazepin | 164 - 165 |
| e) 1,2,3,4,4a,5-Hexahydro-3,10-dimethyl-7-(2'-chlorphenyl)-pyrazino[1,2-a][1,4]-benzodiazepin | 145 - 147 |
| f) 1,2,3,4,4a,5-Hexahydro-9-brom-3,10-dimethyl-7-(2'-chlorphenyl)-pyrazino[1,2-a]-[1,4]benzodiazepin | 106 - 108 |
| g) 1,2,3,4,4a,5-Hexahydro-3,9,10-trimethyl-7-phenyl-pyrazino[1,2-a][1,4]benzodiazepin | 139 - 140 |
| h) 1,2,3,4,4a,5-Hexahydro-3-methyl-7-phenyl-pyrazino[1,2-a][1,4]benzodiazepin | 111 - 114 |
| i) 1,2,3,4,4a,5-Hexahydro-9-fluor-3-methyl-7-phenyl-pyrazino[1,2-a][1,4]benzodiazepin-dihydrochlorid . 0,3 Mol Äthanol | 200 |
| k) 1,2,3,4,4a,5-Hexahydro-9-nitro-3-methyl-7-phenyl-pyrazino[1,2-a][1,4]benzodiazepin | 191 - 194 |
| l) 1,2,3,4,4a,5-Hexahydro-9,10-dichlor-3-methyl-7-phenyl-pyrazino[1,2-a][1,4]benzo-diazepin | 158 - 160 |
| m) 1,2,3,4,4a,5-Hexahydro-10-methoxy-3-methyl-7-phenyl-pyrazino[1,2-a][1,4]benzodiazepin | 121 - 123 |
| n) 1,2,3,4,4a,5-Hexahydro-3-methyl-7-(2'-chlorphenyl)-pyrazino[1,2-a][1,4]benzodiazepin | 136 - 138 |

Beispiel 7

3,7 g 7-Chlor-1-(β-chloräthyl)-2-chlormethyl-5-phenyl-2,3-dihydro-1H-1,4-benzodiazepin werden in 50 ml Benzylamin 5,5 h auf 120 °C erhitzt. Anschließend wird das überschüssige Benzylamin im Vakuum abgezogen, das Reaktionsprodukt mit verdünnter Natriumhydroxidlösung versetzt und aus Chloroform isoliert. Das aus Äthanol umkristallisierte 1,2,3,4,4a,5-Hexahydro-9-chlor-3-benzyl-7-phenyl-pyrazino[1,2-a][1,4]benzodiazepin-dihydrochlorid . 1 Mol $H_2O$ . 1 Mol Äthanol hat einen Fp. von 236-239 °C (Zersetzung). Ausbeute 3 g.

Entsprechend erhält man aus 7-Chlor-1-(β-chloräthyl)-2-chlormethyl-5-phenyl-2,3-dihydro-1H-1,4-benzodiazepin mit β-Methoxyäthylamin, 1,3-Aminopropanol, Phenäthylamin, Isopentylamin und Neopentylamin die folgenden Verbindungen :

| | Fp. °C |
|---|---|
| 1,2,3,4,4a,5-Hexahydro-9-chlor-3-(ß-methoxyäthyl)-7-phenyl-pyrazino[1,2-a][1,4]benzodiazepindihydrochlorid . 0,7 Mol $H_2O$ | 237-240 |
| 1,2,3,4,4a,5-Hexahydro-9-chlor-3-(γ-hydroxypropyl)-7-phenyl-pyrazino[1,2-a][1,4]benzodiazepindihydrochlorid . 1 Mol $H_2O$ | 205 |
| 1,2,3,4,4a,5-Hexahydro-9-chlor-3-(phenäthyl)-7-phenyl-pyrazino[1,2-a][1,4]benzodiazepin | 119-121 |
| 1,2,3,4,4a,5-Hexahydro-9-chlor-3-isopentyl-7-phenyl-pyrazino[1,2-a][1,4]benzodiazepin-dihydrochlorid . 0,5 Mol $H_2O$ . 1 Mol Äthanol | 240-245 |
| 1,2,3,4,4a,5-Hexahydro-9-chlor-3-neopentyl-7-phenyl-pyrazino[1,2-a][1,4]benzodiazepin-dihydrochlorid . 0,3 Mol $H_2O$ . 1 Mol Äthanol | 215 |

Beispiel 8

4,0 g 1,2,3,4,4a,5-Hexahydro-9-chlor-3-(β-hydroxyäthyl)-7-phenyl-pyrazino[1,2-a][1,4]benzodiazepin-dihydrochlorid·2,5 Mol $H_2O$ werden mit 15 ml Thionylchlorid 20 min. unter Rückfluß erhitzt. Das Lösungsmittel wird anschließend abgezogen, die Base mit verdünnter wäßriger Natriumhydroxidlösung unter Eiskühlung freigesetzt und aus Chloroform isoliert. Durch Umkristallisation aus Isopropanol werden 3,2 g 1,2,3,4,4a,5-Hexahydro-9-chlor-3-(β-chloräthyl)-7-phenyl-pyrazino[1,2-a][1,4]benzodiazepin-dihydrochlorid . 1,0 Mol $H_2O$ · 0,6 Mol Isopropanol mit Fp. 220 °C (Zersetzung) erhalten.

Beispiel 9

5,5 g 1,2,4,4a-Tetrahydro-7-(2'-chlorphenyl)-5H-[1,4]oxazino[4,3-a][1,4]benzodiazepin werden in 45 ml Acetanhydrid gelöst und bei 35 °C mit 5,14 g Kupfer(II)-nitrat-trihydrat portionsweise versetzt. Die Temperatur wird 1,5 h belassen, die Reaktionslösung anschließend auf Eis gegossen. Nach Verzetzen mit wäßriger verdünnter Natriumhydroxidlösung und Ammoniaklösung wird das Reaktionsprodukt aus Chloroform isoliert. Nach Säulenchromatographie an Aluminiumoxid der Aktivitätsstufe II mit Methylenchlorid/Chloroform werden aus Äther 2,0 g kristallisiertes 1,2,4,4a-Tetrahydro-9-nitro-7-(2'-chlorphenyl)-5H[1,4]oxazino[4,3-a][1,4]benzodiazepin vom Fp. 164-166 °C und 1,2 g isomeres 1,2,4,4a-Tetrahydro-11-nitro-7-(2'-chlorphenyl)-5H[1,4]oxazino[4,3-a][1,4]-benzodiazepin als Öl erhalten. IR: 1 595, 1 615 $cm^{-1}$ (C = C, C = N).

Entsprechend erhält man aus dem 5-Phenyl substituierten Derivat 1,2,4,4a-Tetrahydro-9-nitro-7-phenyl-5H[1,4]oxazino[4,3-a][1,4]benzodiazepin mit Fp. 142-145 °C und 1,2,4,4a-Tetrahydro-11-nitro-7-phenyl-5H[1,4]oxazino[4,3-a][1,4]benzodiazepin mit Fp. 184-186 °C.

Beispiel 10

4,9 g 1,2,3,4,4a,5-Hexahydro-9-chlor-3-(β-chloräthyl)-7-phenyl-pyrazino[1,2-a][1,4]benzodiazepin-di-hydrochlorid . 1,0 Mol $H_2O$ . 0,6 Mol Isopropanol werden zu einer Lösung von 0,75 g Natrium in 70 ml Methanol gegeben und 5 h unter Rückfluß erhitzt. Der nach Abziehen des Lösungsmittels verbleibende Rückstand wird in Chloroform gelöst, die organische Phase wird mit Wasser gewaschen. Nach Abziehen des Lösungsmittels erhält man 3,3 g 1,2,3,4,4a,5-Hexahydro-9-chlor-3-(β-methoxyäthyl)-7-phenyl-pyrazino[1,2-a][1,4]benzodiazepin. Die Substanz kristallisiert als Dihydrochlorid mit 0,7 Mol Wasser mit Fp. 237-240 °C aus Äthanol.

Beispiel 11

4,0 g 1,2,3,4,4a,5-Hexahydro-9-chlor-3-(β-hydroxyäthyl)-7-phenyl-pyrazino[1,2-a][1,4]benzodiazepin-dihydrochlorid . 2,5 Mol $H_2O$ werden zu 0,8 g Natriumhybrid in 100 ml Dimethylformamid gegeben. Nach Zugabe von 1,5 g Methyljodid wird die Reaktionslösung 4 h bei Raumtemperatur belassen. Das nach Abziehen des Lösungsmittels erhaltene Rohprodukt wird durch Chromatographie an Aluminiumoxid der Aktivitätsstufe II mit Chloroform gereinigt. Man erhält 1,7 g 1,2,3,4,4a,5-Hexahydro-9-chlor-3-(β-methoxy-äthyl)-7-phenyl-pyrazino[1,2-a][1,4]benzodiazepin. Die Substanz kristallisiert als Dihydrochlorid mit 0,7 Mol Wasser mit Fp. 237-240 °C aus Äthanol.

Beispiel 12

10,0 g 1,2,4,4a-Tetrahydro-5-(2'-fluorphenyl)-5H[1,4]oxazino[4,3-a][1,4]benzodiazepin werden in 150 ml Methylenchlorid mit 6,0 g N-bromsuccinimid 3,5 h unter Rückfluß erhitzt. Nach üblicher Aufarbeitung erhält man 4,0 g öliges 1,2,4,4a-Tetrahydro-9-brom-7-(2'-fluorphenyl)-5H[1,4]oxazino[4,3-a][1,4]-benzodiazepin ; IR : 1 610 $cm^{-1}$.

Beispiel 13

Kapseln mit 100 mg 1,2,4,4a-Tetrahydro-9-chlor-7-phenyl-5H[1,4]thiazino[4,3-a][1,4]benzodiazepin als Wirkstoff. 1 Kapsel enthält :

| | |
|---|---|
| Wirkstoff | 100 mg |
| Lactose | 90 mg |
| Aerosil 200 | 4 mg |
| Talkum | 4 mg |
| Magnesiumstearat | 2 mg |
| | 200 mg |

Herstellungsverfahren : Der Wirkstoff wird mit den Hilfsstoffen vermischt und in Kapseln der Größe 2 abgefüllt.

Zusammen mit den genannten Hilfsstoffen können aus den übrigen Wirkstoffen Kapseln mit 100 mg Wirkstoffgehalt hergestellt werden.

Beispiel 14

Tabletten mit 50 mg 1,2,4,4a-Tetrahydro-9-chlor-7-phenyl-5H[1,4]oxazino[4,3-a][1,4]benzodiazepin-maleinat als Wirkstoff.

1 Tablette enthält :

| | |
|---|---|
| Wirkstoff | 50 mg |
| Lactose | 60 mg |
| Maisstärke | 30 mg |
| Primojel | 4 mg |
| Gelatine | 2 mg |
| Aerosil 200 | 2 mg |
| Magnesiumstearat | 2 mg |
| | 150 mg |

Herstellungsverfahren : Aus der Gelatine wird in Wasser ein 10 %-iger Schleim hergestellt. Wirkstoff, Lactose, Maisstärke und Primojel werden gemischt und mit dem vorstehend hergestellten Schleim durch ein Sieb der Maschenweite 1,5 mm granuliert. Das Granulat wird bei 40 °C getrocknet, nochmals durch das Sieb passiert, mit Aerosil 200 und Magnesiumstearat vermischt und zu Tabletten verpreßt. Stempel 9 mm. In gleicher Weise können die übrigen Wirkstoffe zu Tabletten verarbeitet werden.

Beispiel 15

Dragées mit 50 mg 1,2,3,4,4a,5-Hexahydro-3,9-dimethyl-7-phenyl-pyrazino[1,2-a][1,4]benzodiazepin-dihydrochlorid . 1,5 Mol $H_2O$ als Wirkstoff.

Die wie in Beispiel 14 hergestellten Tabletten werden nach bekannten Verfahren mit einer Hülle überzogen. Die fertigen Dragées werden mit Hilfe von Bienenwachs poliert.

**Ansprüche**

1. [1,2]-Anellierte 7-Phenyl-1,4-benzodiazepine der allgemeinen Formel I

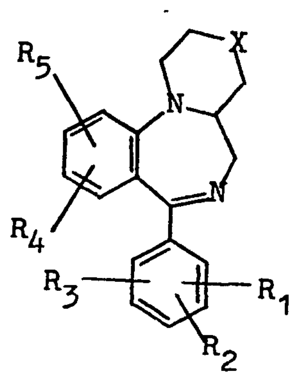

(I)

worin X ein Sauerstoffatom, ein Schwefelatom oder eine Iminogruppe NR ist, in welcher R ein Wasserstoffatom, ein $C_1$-$C_5$-Alkylrest, ggf. endständig substituiert mit einem Phenylrest, der durch 1 oder 2 Methoxy oder durch 3,4-Methylen- oder 3,4-Äthylendioxy substituiert sein kann, ein $C_2$-$C_5$-Alkyl, endständig substituiert mit Halogen, Hydroxy oder Methoxy, oder ein $C_3$-$C_5$-Alkenylrest ist, $R_1$ bis $R_5$ gleich oder verschieden sind und ein Wasserstoffatom, ein Halogenatom, eine Trifluormethylgruppe, eine Nitrogruppe, eine Alkylgruppe, eine Alkoxygruppe oder eine Alkylthiogruppe bedeuten, wobei der Alkylrest jeweils 1 bis 4 C-Atome haben kann, oder zwei benachbarte Reste eine Methylen- oder Äthylendioxygruppe sind sowie deren Säureadditionssalze.

2. [1,2]-Anellierte 7-Phenyl-1,4-benzodiazepine entsprechend Anspruch 1, dadurch gekennzeichnet, daß sich $R_4$ und/oder $R_5$ in 9- und/oder 10-Stellung befinden und unabhängig voneinander jeweils vorzugsweise Wasserstoff, Chlor, Brom oder Methyl sind, für Fluor, Nitro und Trifluormethyl Monosubstitution in 9-Stellung und für Methoxy und Methylthio Monosubstitution in 10-Stellung bevorzugt sind bzw. $R_4$ und $R_5$ zusammen Methylendioxy oder Äthylendioxy bedeuten.

3. [1,2]-Anellierte 7-Phenyl-1,4-benzodiazepine entsprechend Anspruch 1, dadurch gekennzeichnet, daß der 7-Phenylrest unsubstituiert oder substituiert durch Trifluormethyl, Methylendioxy, Äthylendioxy oder durch 1 bis 3 gleiche oder verschiedene Substituenten aus der Reihe Halogen, Methyl und Methoxy ist.

4. [1,2]-Anellierte 7-Phenyl-1,4-benzodiazepine entsprechend Ansprüchen 2 und 3, dadurch gekennzeichnet, daß X ein Sauerstoffatom, ein Schwefelatom oder eine Iminogruppe NR ist, in welcher R ein Wasserstoffatom, ein $C_1$- bis $C_5$-Alkylrest, ein $C_1$- oder $C_2$-Alkylrest, endständig substituiert mit einem Phenylrest, ein $C_2$- oder $C_3$-Alkylrest, endständig substituiert mit Chlor, Hydroxy oder Methoxy, oder ein Allylrest ist.

5. 1,2,4,4a-Tetrahydro-A-7-phenyl-5H[1,4]oxazino[4,3-a][1,4]benzodiazepin, worin A Wasserstoff, 9-Fluor, 9-Chlor, 9-Brom, 9-Nitro, 11-Nitro, 9-Methyl, 10-Methyl, 9,10-Dimethyl, 9,10-Dichlor, 10-Chlor-9-methyl, 10-Methoxy-, 9,10-Methylendioxy, 9-Brom-10-methyl oder 9-Trifluormethyl bedeutet.

6. 1,2,4,4a-Tetrahydro-A-7-(B-phenyl)-5H[1,4]oxazino[4,3-a][1,4]benzodiazepin, worin
A Wasserstoff, 9-Chlor, 9-Nitro oder 11-Nitro und B 2'-Chlor,
A Wasserstoff oder 9-Brom und B 2'-Fluor,
A 9-Chlor und B 3'-Trifluormethyl oder 3',4'-Dichlor oder
A 9-Fluor und B 3',4',5'-Trimethoxy bedeutet.

7. 1,2,4,4a-Tetrahydro-A-7-(B-phenyl)-5H[1,4]thiazino[4,3-a][1,4]benzodiazepin, worin

A Wasserstoff, 9-Chlor, 9-Fluor, 9-Nitro, 9-Methyl oder 10-Methoxy und B Wasserstoff,
A 9-Chlor und B 3'-Trifluormethyl oder
A Wasserstoff und B 2'-Fluor bedeutet.

8. 1,2,3,4,4a,5-Hexahydro-9-chlor-3C-7-phenyl-pyrazino[1,2-a][1,4]benzodiazepin, worin C Wasserstoff, Methyl, Isopropyl, Butyl, tert.-Butyl, Isopentyl-, Neopentyl-, Allyl, β-Hydroxyäthyl, β-Chloräthyl, β-Methoxyäthyl, 3-Hydroxypropyl, Benzyl, Phenäthyl oder 3,4-Dimethoxyphenäthyl bedeutet.

9. 1,2,3,4,4a,5-Hexahydro-A-3-methyl-7-(B-phenyl)-pyrazino[1,2-a][1,4]benzodiazepin, worin
A Wasserstoff, 9-Fluor, 9-Nitro, 10-Methoxy, 9-Methyl, 10-Methyl, 9,10-Dimethyl oder 9,10-Dichlor und B Wasserstoff,
A Wasserstoff, 9-Chlor, 10-Methyl, oder 9-Brom- 10-methyl und B 2'-Chlor oder
A 9-Chlor und B 2'-Methoxy bedeutet.

10. 1,2,3,4,4a,5-Hexahydro-A-7-(B-phenyl)-pyrazino[1,2-a][1,4]benzodiazepin, worin
A Wasserstoff, 9-Brom, 9-Methyl, 9,10-Dimethyl, 10-Methoxy oder 9-Brom-10-methyl und B Wasserstoff,
A 9-Chlor und B 2'-Chlor oder 3',4'-Dichlor oder
A 9-Brom und B 3',4'-Dichlor bedeutet.

11. Verfahren zur Herstellung von [1,2]-anellierten 7-Phenyl-1,4-benzodiazepinen der allgemeinen Formel I

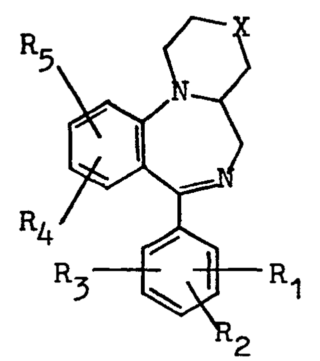

(I)

worin X ein Sauerstoffatom, ein Schwefelatom oder eine Iminogruppe NR ist, in welcher R ein Wasserstoffatom, ein $C_1$-$C_5$-Alkylrest, ggf. endständig substituiert mit einem Phenylrest, der durch 1 oder 2 Methoxy oder durch 3,4-Methylen- oder 3,4-Äthylendioxy substituiert sein kann, ein $C_2$-$C_5$-Alkyl, endständig substituiert mit Halogen, Hydroxy oder Methoxy, oder ein $C_3$-$C_5$-Alkenylrest ist,

$R_1$ bis $R_5$ gleich oder verschieden sind und ein Wasserstoffatom, ein Halogenatom, eine Trifluormethylgruppe, eine Nitrogruppe, eine Alkylgruppe, eine Alkoxygruppe oder eine Alkylthiogruppe bedeuten, wobei der Alkylrest jeweils 1 bis 4 C-Atome haben kann, oder zwei benachbarte Reste eine Methylen- oder Äthylendioxygruppe sind, sowie deren Säure-additionssalze, dadurch gekennzeichnet, daß man Verbindungen der allgemeinen Formel II

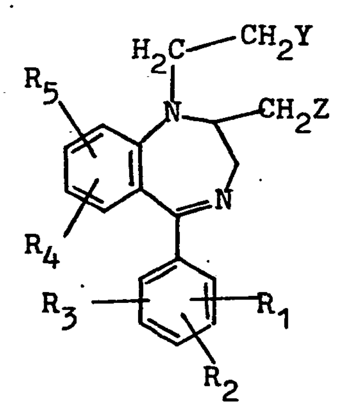

(II)

in welcher $R_1$ bis $R_5$ bis obige Bedeutung haben, und Y und Z gleich oder verschieden sind und einen reaktiven Rest darstellen, so ein Halogen, insbesondere Chlor- oder Brom, einen Toluolsulfonyloxy, Benzolsulfonyloxy- oder Methansulfonyloxyrest, in Gegenwart eines Alkalimetall- oder Erdalkalimethallhydroxids, Alkalimetall- oder Erdalkalimetallcarbonats, Alkalimetall- oder Erdalkalimetallsulfids oder

einer Aminoverbindung $RNH_2$, wobei R die obige Bedeutung hat, oder des entsprechenden Alkalimetallamids bei Temperaturen zwischen 60 und 120 °C, ggf. in Gegenwart eines inerten Lösungsmittels cyclisiert und ggf. die gebildete Base in das Säureadditionssalz überführt oder aus dem Säureadditionssalz die freie Base isoliert.

12. Verfahren nach Anspruch 11, dadurch gekennzeichnet, daß die reaktiven Reste Y und Z gleiche Bedeutung haben und Chlor oder Toluolsulfonyloxy bedeuten.

13. Arzneimittel, bestehend aus einer oder mehreren Verbindungen gemäß Anspruch 1 und üblichen Träger- und Hilfssubstanzen.

**Claims**

1. [1,2]-Fused 7-phenyl-1,4-benzodiazepines of the general formula I

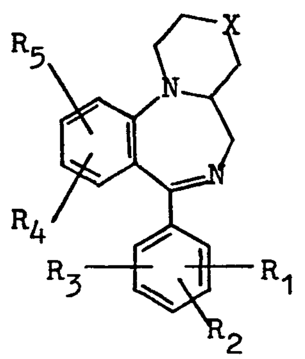

(I)

wherein X is an oxygen atom, a sulphur atom or an amino group NR in which R is a hydrogen atom, a $C_1$-$C_5$ alkyl radical which is optionally substituted in the terminal position by a phenyl radical which can be mono- or di-substituted by methoxy or substituted by 3,4-methylenedioxy or 3,4-ethylenedioxy, a $C_2$-$C_5$ alkyl which is substituted in the terminal position by halogen, hydroxyl or methoxy, or a $C_3$-$C_5$ alkenyl radical, and $R_1$ to $R_5$ are identical or different and denote a hydrogen atom, a halogen atom, a trifluoromethyl group, a nitro group, an alkyl group, an alkoxy group or an alkylthio group, where each of the alkyl radicals can have 1 to 4 C atoms, or two adjacent radicals are a methylenedioxy or ethylenedioxy group, and their acid addition salts.

2. [1,2]-Fused 7-phenyl-1,4-benzodiazepines according to Claim 1, characterised in that $R_4$ and/or $R_5$ are in the 9-position and/or the 10-position and each are, independently of one another, preferably hydrogen, chlorine, bromine or methyl, monosubstitution in the 9-position being preferred for fluorine, nitro and trifluoromethyl and monosubstitution in the 10-position being preferred in the case of methoxy and methylthio, or $R_4$ and $R_5$ together denote methylenedioxy or ethylenedioxy.

3. [1,2]-Fused 7-phenyl-1,4-benzodiazepines according to Claim 1, characterised in that the 7-phenyl radical is unsubstituted or substituted by trifluoromethyl, methylenedioxy, ethylenedioxy or by 1 to 3 identical or different substituents from the group comprising halogen, methyl and methoxy.

4. [1,2]-Fused 7-phenyl-1,4-benzodiazepines according to Claims 2 and 3, characterised in that X is an oxygen atom, a sulphur atom or an imino group NR in which R is a hydrogen atom, a $C_1$ to $C_5$ alkyl radical, a $C_1$ or $C_2$ alkyl radical which is substituted in the terminal position by a phenyl radical, a $C_2$ or $C_3$ alkyl radical which is substituted in the terminal position by chlorine, hydroxyl or methoxy, or an allyl radical.

5. 1,2,4,4a-Tetrahydro-A-7-phenyl-5H[1,4]oxazino[4,3-a][1,4]benzodiazepine, wherein A denotes hydrogen, 9-fluoro, 9-chloro, 9-bromo, 9-nitro, 11-nitro, 9-methyl, 10-methyl, 9,10-dimethyl, 9,10-dichloro, 10-chloro-9-methyl, 10-methoxy, 9,10-methylenedioxy, 9-bromo-10-methyl or 9-trifluoromethyl.

6. 1,2,4,4a-Tetrahydro-A-7(B-phenyl)-5H[1,4]oxazino[4,3-a][1,4]benzodiazepine, wherein
A denotes hydrogen, 9-chloro, 9-nitro or 11-nitro and B denotes 2′-chloro,
A denotes hydrogen or 9-bromo and B denotes 2′-fluoro,
A denotes 9-chloro and B denotes 3′-trifluoromethyl or 3′,4′-dichloro or
A denotes 9-fluoro and B denotes 3′,4′,5′-trimethoxy.

7. 1,2,4,4a-Tetrahydro-A-7-(B-phenyl)-5H[1,4]thiazino[4,3-a][1,4]benzodiazepine, wherein
A denotes hydrogen, 9-chloro, 9-fluoro, 9-nitro, 9-methyl of 10-methoxy and B denotes hydrogen,
A denotes 9-chloro and B denotes 3′-trifluoromethyl or
A denotes hydrogen and B denotes 2′-fluoro.

8. 1,2,3,4,4a-5-Hexahydro-9-chloro-3C-7-phenylpyrazino[1,2-a][1,4]benzodiazepine, wherein C denotes hydrogen, methyl, isopropyl, butyl, tert.-butyl, isopentyl, neopentyl, allyl, β-hydroxyethyl, β-chloroethyl, β-methoxyethyl, 3-hydroxypropyl, benzyl, phenethyl or 3,4-dimethoxyphenethyl.

9. 1,2,3,4,4a,5-Hexahydro-A-3-methyl-7-(B-phenyl)-pyrazino[1,2-a][1,4]benzodiazepine, wherein

A denotes hydrogen, 9-fluoro, 9-nitro, 10-methoxy, 9-methyl, 10-methyl, 9,10-dimethyl or 9,10-dichloro and B denotes hydrogen,

A denotes hydrogen, 9-chloro, 10-methyl or 9-bromo-10-methyl and B denotes 2'-chloro or

A denotes 9-chloro and B denotes 2'-methoxy.

10. 1,2,3,4,4a,5-Hexahydro-A-7-(B-phenyl)-pyrazino[1,2-a][1,4]benzodiazepine, wherein

A denotes hydrogen, 9-bromo, 9-methyl, 9,10-dimethyl, 10-methoxy or 9-bromo-10-methyl and B denotes hydrogen,

A denotes 9-chloro and B denotes 2'-chloro or 3',4'-dichloro or

A denotes 9-bromo and B denotes 3',4'-dichloro.

11. Process for the preparation of [1,2]-fused 7-phenyl-1,4-benzodiazepines of the general formula I

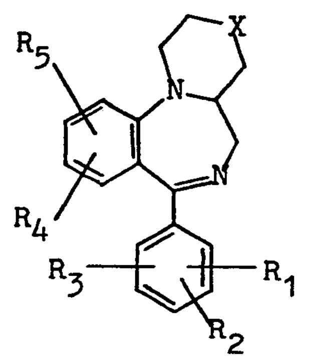

(I)

wherein X is an oxygen atom, a sulphur atom or an imino group NR in which R is a hydrogen atom, a $C_1$-$C_5$ alkyl radical which is optionally substituted in the terminal position by a phenyl radical which can be mono- or disubstituted by methoxy or substituted by 3,4-methylenedioxy or 3,4-ethylenedioxy, a $C_2$-$C_5$ alkyl which is substituted in the terminal position by halogen, hydroxyl or methoxy, or a $C_3$-$C_5$ alkenyl radical, $R_1$ to $R_5$ are identical or different and denote a hydrogen atom, a halogen atom, a trifluoromethyl group, a nitro group, and alkyl group, an alkoxy group or an alkylthio group, where each of the alkyl radicals can have 1 to 4 C atoms, or two adjacent radicals are a methylenedioxy or ethylenedioxy group, and of their acid addition salts, characterised in that compounds of the general formula II

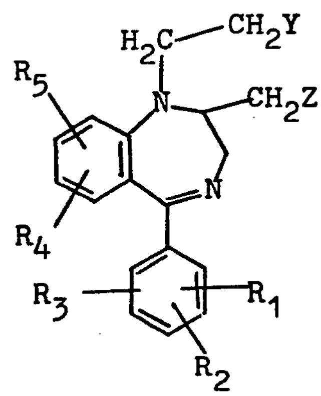

in which $R_1$ to $R_5$ have the above meaning and Y and Z are identical or different and represent a reactive radical, such as a halogen, in particular chlorine or bromine, or a toluenesulphonyloxy, benzenesulphonyloxy or methanesulphonyloxy radical, are cyclised in the present of an alkali metal hydroxide or alkaline earth metal hydroxide, an alkali metal carbonate or alkaline earth metal carbonate, an alkali metal sulphide or alkaline earth metal sulphide or an amino compound $RNH_2$, where R has the above meaning, or the corresponding alkali metal amide at temperatures between 60 and 120 °C, if desired in the presence of an inert solvent, and, if desired, the base formed is converted to the acid addition salt or the free base is isolated from the acid addition salt.

12. Process according to Claim 11, characterised in that the reactive radicals Y and Z have the same meaning and denote chlorine or toluenesulphonyloxy.

13. Medicaments, consisting of one or more compounds according to Claim 1 and of conventional carriers and adjuvants.

**Revendications**

1. 7-phényl-1,4-benzodiazépines condensées en position [1,2] et répondant à la formule générale I

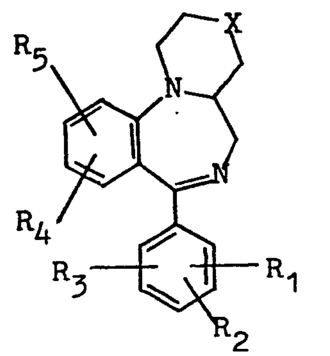

(I)

dans laquelle X représente un atome d'oxygène, un atome de soufre ou un groupe imino NR dans lequel R représente un atome d'hydrogène, un groupe alkyle contenant 1 à 5 atomes de carbone, éventuellement substitué en position terminale par un groupe phényle qui peut être substitué par un ou deux groupes méthoxy ou par un groupe 3,4-méthylène-dioxy ou 3,4-éthylène-dioxy, un groupe alkyle contenant 2 à 5 atomes de carbone, substitué en position terminale par un atome d'halogène, un groupe hydroxy ou un groupe méthoxy, ou encore un groupe alcényle contenant 3 à 5 atomes de carbone, les radicaux $R_1$ à $R_5$ sont identiques ou différents et représentent chacun un atome d'hydrogène, un atome d'halogène, un groupe trifluorométhyle, un groupe nitro, un groupe alkyle, un groupe alcoxy ou un groupe alkylthio, chaque groupe alkyle pouvant contenir 1 à 4 atomes de carbone, ou deux radicaux voisins sont un groupe méthylène-dioxy ou un groupe éthylène-dioxy, de même que leurs sels d'addition d'acide.

2. 7-phényl-1,4-benzodiazépines condensées en position [1,2] suivant la revendication 1, caractérisées en ce que les radicaux $R_4$ et/ou $R_5$ occupent la position 9 et/ou la position 10 et représentent chacun indépendamment l'un de l'autre, de préférence, un atome d'hydrogène, un atome de chlore, un atome de brome ou un groupe méthyle, la monosubstitution en position 9 étant préférée pour l'atome de fluor, le groupe nitro et le groupe trifluorométhyle, tandis que la monosubstitution en position 10 est préférée par le groupe méthoxy et le groupe méthylthio, ou encore $R_4$ et $R_5$ ensemble représentent un groupe méthylène-dioxy ou un groupe éthylène-dioxy.

3. 7-phényl-1,4-benzodiazépines condensées en position [1,2] suivant la revendication 1, caractérisées en ce que le groupe 7-phényle est non substitué ou substitué par un groupe trifluorométhyle, un groupe méthylène-dioxy, un groupe éthylène-dioxy ou par un à trois substituants identiques ou différents choisis parmi la série comprenant un atome d'halogène, un groupe méthyle et un groupe méthoxy.

4. 7-phényl-1,4-benzodiazépines condensées en position [1,2] suivant les revendications 2 et 3, caractérisées en ce que X représente un atome d'oxygène, un atome de soufre ou un groupe imino NR dans lequel R est un atome d'hydrogène, un groupe alkyle contenant 1 à 5 atomes de carbone, un groupe alkyle contenant 1 ou 2 atomes de carbone et substitué en position terminale par un groupe phényle, un groupe alkyle contenant 2 ou 3 atomes de carbone et substitué en position terminale par un atome de chlore, un groupe hydroxy ou un groupe méthoxy, ou encore un groupe allyle.

5. 1,2,4,4a-tétrahydro-A-7-phényl-5H[1,4]oxazino[4,3-a][1,4]benzodiazépine dans laquelle A représente un atome d'hydrogène, un groupe 9-fluoro, 9-chloro, 9-bromo, 9-nitro, 11-nitro, 9-méthyle, 10-méthyle, 9,10-diméthyle, 9,10-dichloro, 10-chloro- 9-méthyle, 10-méthoxy, 9,10-méthylène-dioxy, 9-bromo- 10-méthyle ou 9-trifluorométhyle.

6. 1,2,4,4a-tétrahydro-A-7-(B-phényl)-5H[1,4]oxazino[4,3-a][1,4]benzodiazépine dans laquelle A représente un atome d'hydrogène, un groupe 9-chloro, 9-nitro ou 11-nitro et B représente un groupe 2'-chloro,

A représente un atome d'hydrogène ou un groupe 9-bromo et B représente un groupe 2'-fluoro,

A représente un groupe 9-chloro et B représente un groupe 3'-trifluorométhyle ou 3',4'-dichloro, ou encore

A représente un groupe 9-fluoro et B représente un groupe 3',4',5'-triméthoxy.

7. 1,2,4,4a-tétrahydro-A-7-(B-phényl)-5H[1,4]thiazino[4,3-a][1,4]benzodiazépine, dans laquelle

A représente un atome d'hydrogène, un groupe 9-chloro, 9-fluoro, 9-nitro, 9-méthyle ou 10-méthoxy et B représente un atome d'hydrogène,

A représente un groupe 9-chloro et B représnnte un groupe 3'-trifluorométhyle, ou

A représente un atome d'hydrogène et B représente un groupe 2'-fluoro.

8. 1,2,3,4,4a,5-hexahydro-9-chloro-3C-7-phényl-pyrazino[1,2-a][1,4]benzodiazépine, dans laquelle C représente un atome d'hydrogène, un groupe méthyle, un groupe isopropyle, un groupe butyle, un groupe tert-butyle, un groupe isopentyle, un groupe néopentyle, un groupe allyle, un groupe β-hydroxyéthyle, un groupe β-chloréthyle, un groupe β-méthoxyéthyle, un groupe 3-hydroxypropyle, un groupe benzyle, un groupe phénéthyle ou un groupe 3,4-diméthoxyphénéthyle.

9. 1,2,3,4,4a,5-hexahydro-A-3-méthyl-7-(B-phényl)-pyrazino[1,2-a][1,4]benzodiazépine, dans laquelle

A représente un atome d'hydrogène, un groupe 9-fluoro, 9-nitro, 10-méthoxy, 9-méthyle, 10-méthyle, 9,10-diméthyle ou 9,10-dichloro et B représente un atome d'hydrogène,

A représente un atome d'hydrogène, un groupe 9-chloro, un groupe 10-méthyle ou un groupe 9-bromo-10-méthyle et B représente un groupe 2'-chloro, ou

A représente un groupe 9-chloro et B représente un groupe 2'-méthoxy.

10. 1,2,3,4,4a,5-hexahydro-A-7-(B-phényl)-pyrazino[1,2-a][1,4]benzodiazépine, dans laquelle A représente un atome d'hydrogène, un groupe 9-bromo, un groupe 9-méthyle, un groupe 9,10-diméthyle, un groupe 10-méthoxy ou un groupe 9-bromo-10-méthyle et B représente un atome d'hydrogène,

A représente un groupe 9-chloro et B représente un groupe 2'-chloro ou un groupe 3',4'-dichloro ou encore A représente un groupe 9-bromo et B représente un groupe 3',4'-dichloro.

11. Procédé de préparation de 7-phényl-1,4-benzodiazépines condensées en position [1,2] et répondant à la formule générale I :

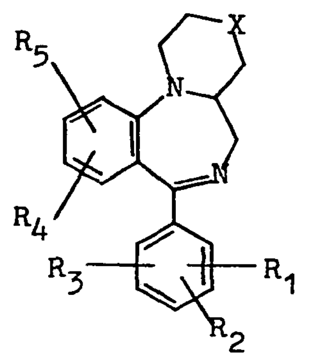

(I)

dans laquelle X représente un atome d'oxygène, un atome de soufre ou un groupe imino NR dans lequel R est un atome d'hydrogène, un groupe alkyle contenant 1 à 5 atomes de carbone, éventuellement substitué en position terminale par un groupe phényle qui peut être substitué par un ou deux groupes méthoxy, par un groupe 3,4-méthylène-dioxy ou par un groupe 3,4-éthylènedioxy, un groupe alkyle contenant 2 à 5 atomes de carbone, substitué en position terminale par un atome d'halogène, un groupe hydroxy ou un groupe méthoxy, ou un groupe alcényle contenant 3 à 5 atomes de carbone,

les radicaux $R_1$ à $R_5$ sont identiques ou différents et représentent chacun un atome d'hydrogène, un atome d'halogène, un groupe trifluorométhyle, un groupe nitro, un groupe alkyle, un groupe alcoxy ou un groupe alkylthion

chaque groupe alkyle pouvant contenir 1 à 4 atomes de carbone, ou encore deux radicaux voisins sont un groupe méthylène-dioxy ou un groupe éthylène-dioxy, ainsi que de leurs sels d'addition d'acide, caractérisé en ce qu'on cyclise des composés de formule générale II :

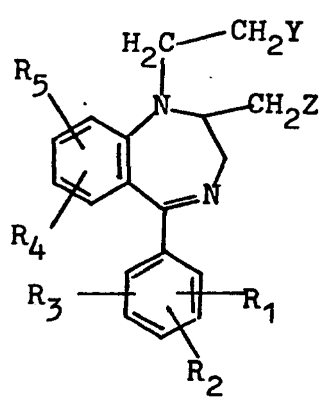

(II)

dans laquelle les radicaux $R_1$ à $R_5$ ont les significations indiquées ci-dessus, tandis que Y et Z sont identiques ou différents et représentent chacun un radical réactif, soit un atome d'halogène, en particulier, un atome de chlore ou un atome de brome, un groupe toluène-sulfonyloxy, un groupe benzène-sulfonyloxy ou un groupe méthane-sulfonyloxy, en présence d'un hydroxyde d'un métal alcalin ou alcalino-terreux, d'un carbonate d'un métal alcalin ou alcalino-terreux, d'un sulfure d'un métal alcalin ou alcalino-terreux, ou d'un composé aminé $RNH_2$, R ayant les significations indiquées ci-dessus, ou en présence de l'amidure de métal alcalin correspondant à des températures comprises entre 60 et 120 °C, éventuellement en présence d'un solvant inerte, puis on transforme éventuellement la base formée en un sel d'addition d'acide ou on isole la base libre de ce sel d'addition d'acide.

12. Procédé suivant la revendication 11, caractérisé en ce que les radicaux réactifs Y et Z ont les mêmes significations et représentent un atome de chlore ou un groupe toluène-sulfonyloxy.

13. Médicament constitué d'un ou de plusieurs composés suivant la revendication 1, ainsi que de substances supports et de substances auxiliaires habituelles.